(19) Europäisches Patentamt

European Patent Office

Office européen des brevets

(11) **EP 1 248 575 B1**

(12) **FASCICULE DE BREVET EUROPEEN**

(45) Date de publication et mention
de la délivrance du brevet:
**28.04.2004 Bulletin 2004/18**

(21) Numéro de dépôt: **01903965.0**

(22) Date de dépôt: **17.01.2001**

(51) Int Cl.⁷: **A61B 17/70**

(86) Numéro de dépôt international:
**PCT/FR2001/000136**

(87) Numéro de publication internationale:
**WO 2001/052756 (26.07.2001 Gazette 2001/30)**

(54) **FIXATEUR POUR OS, NOTAMMENT RACHIDIEN**

KNOCHENFIXATEUR, INSBESONDERE FÜR DIE WIRBELSÄULE

BONE FIXATION, IN PARTICULAR SPINAL

(84) Etats contractants désignés:
**AT BE CH CY DE DK ES FI FR GB GR IE IT LI LU
MC NL PT SE TR**

(30) Priorité: **20.01.2000 FR 0000667**

(43) Date de publication de la demande:
**16.10.2002 Bulletin 2002/42**

(73) Titulaire: **Item-Arex
60100 Creil (FR)**

(72) Inventeurs:
• **AUFAURE, Pascal
F-60300 Saint Jean au Bois (FR)**

• **BENOIST, Michel
F-75116 Paris (FR)**
• **DEBURGE, Alain
F-75116 Paris (FR)**
• **ROUX, Didier
F-95270 Asnières sur Oise (FR)**

(74) Mandataire: **Flavenot, Bernard
Société ABRITT
17, rue du Docteur Charcot,
La Norville
91290 Arpajon (FR)**

(56) Documents cités:
**WO-A-95/32675      FR-A- 2 720 261**

## Description

La présente invention concerne les systèmes permettant de maintenir au moins deux portions d'os l'une par rapport à l'autre et leur application à la réalisation d'une ostéosynthèse rachidienne.

Pour réaliser, par exemple, une ostéosynthèse rachidienne, c'est-à-dire une ostéosynthèse entre deux ou plusieurs vertèbres, il est absolument nécessaire que celles-ci soient parfaitement maintenues à une distance constante l'une de l'autre et qu'il ne puisse se produire aucun déplacement de l'une par rapport à l'autre.

On connaît déjà différents systèmes permettant d'obtenir ce résultat, par exemple celui qui est décrit dans le FR-A-2 720 261 intitulé "Implant pour dispositif d'ostéosynthèse".

Cet implant comprend une partie destinée à l'ancrage osseux et une tête la surmontant sur laquelle est fixée au moins une broche de liaison entre deux implants successifs, la tête de l'implant comportant des moyens de réception, d'immobilisation et d'orientation angulaire de la broche de liaison constitués par deux mors formés par une coupelle inférieure et une coupelle supérieure complémentaires, chacune de celles-ci comportant au moins une demi-gorge formant au moins une gorge cylindrique, lesquelles coupelles sont montées librement rotatives par l'intermédiaire d'un trou sur un prolongement extérieur axial de la partie d'ancrage, et entre lesquelles est susceptible d'être insérée au moins une broche de liaison dont l'immobilisation après une orientation angulaire choisie s'effectue par l'intermédiaire d'un écrou de serrage disposé à l'extrémité libre du prolongement de la partie d'ancrage et agissant en blocage contre lesdites coupelles prenant appui sur un épaulement ménagé au pied dudit prolongement.

Cet implant de l'art antérieur comporte en outre une tête destinée à former un double étage, chaque étage inférieur ou supérieur comportant des moyens de réception et d'immobilisation d'au moins deux broches de liaison correspondantes se superposant, chacune d'elles pouvant être orientée de manières différentes ou non puis immobilisée successivement indépendamment l'une de l'autre, et de la partie d'ancrage, le double étage de la tête étant obtenu par l'intermédiaire d'un ensemble triple qui est constitué par une coupelle inférieure constituant le mors de l'étage inférieur et reposant sur l'épaulement et par un écrou de serrage destiné à se visser sur le prolongement fileté de la partie d'ancrage et sur lequel sont serties librement à sa partie inférieure une coupelle constituant l'un des mors de l'étage inférieur et à sa partie supérieure une autre coupelle constituant l'un des mors de l'étage supérieur, ledit écrou se prolongeant à sa partie supérieure par une partie cylindrique filetée qui est destinée à la mise en place et au serrage d'un second écrou sur la partie inférieure duquel est sertie librement une coupelle constituant l'autre mors de l'étage supérieur.

Le système décrit ci-dessus donne de bons résultats, mais il est certainement complexe et relativement encombrant.

Aussi, la présente invention a-t-elle pour but de réaliser un perfectionnement au système permettant d'une façon générale de maintenir au moins deux portions d'os l'une par rapport à l'autre, et notamment au système d'ostéosynthèse rachidienne, du type décrit dans ce document antérieur, permettant notamment de réduire son encombrement, même dans le cas de la réalisation illustrée sur la figure 6 de ce document.

Plus précisément, la présente invention a pour objet un système permettant de maintenir au moins deux portions d'os l'une par rapport à l'autre, comportant :

une première vis comportant une première tige à filetage mécanique et une première tête d'épaulement, ladite première tige étant solidaire de ladite première tête d'épaulement par une première de ses deux extrémités, la longueur de cette première tige étant égale à $l_1$,
des moyens pour ancrer la première vis dans une portion d'os,
un écrou apte à se visser sur la première tige, la hauteur de cet écrou étant égale à $h$,
un orifice réalisé sensiblement dans l'axe de la première tige, ledit orifice débouchant à la seconde extrémité de la première tige et ayant une profondeur égale à $p$, caractérisé par la paroi latérale dudit orifice comportant un filetage mécanique, et
une seconde vis comportant une seconde tige à filetage mécanique complémentaire du filetage mécanique de la paroi latérale de l'orifice et une seconde tête d'épaulement, ladite seconde tige étant solidaire de ladite seconde tête d'épaulement par l'une de ses deux extrémités, la longueur de cette seconde tige étant égale à $l_2$.

Selon une autre caractéristique de la présente invention les valeurs $h$, $l_1$, $l_2$ et $p$ sont liées par les deux relations suivantes :

$$h \leq l_1$$

$$l_2 < p$$

La présente invention a aussi pour objet un système permettant de maintenir au moins deux portions d'os l'une par rapport à l'autre, caractérisé par le fait qu'il comporte :

une première vis comportant une première tige à filetage mécanique et une première tête d'épaulement, ladite première tige étant solidaire de ladite première tête d'épaulement par une première de ses deux extrémités, la longueur de cette première tige étant égale à $l_1$,

des moyens pour ancrer la première vis dans une portion d'os,

un écrou apte à se visser sur la première tige, la hauteur de cet écrou étant égale à **h**,

au moins une première barre de liaison apte à être maintenue par pincement entre l'écrou et la première tête d'épaulement, l'épaisseur de cette première barre de liaison étant égale à $e_1$,

un orifice réalisé sensiblement dans l'axe de la première tige, ledit orifice débouchant à la seconde extrémité de la première tige et ayant une profondeur égale à **p**, la paroi latérale dudit orifice comportant un filetage mécanique,

une seconde vis comportant une seconde tige à filetage mécanique complémentaire du filetage mécanique de la paroi latérale de l'orifice et une seconde tête d'épaulement, ladite seconde tige étant solidaire de ladite seconde tête d'épaulement par l'une de ses deux extrémités, la longueur de cette seconde tige étant égale à $l_2$, et

au moins une seconde barre de liaison apte à être maintenue par pincement entre l'écrou et la seconde tête d'épaulement, l'épaisseur de cette seconde barre de liaison étant égale à $e_2$.

Selon une autre caractéristique de la présente invention, les valeurs **h**, $e_1$, $e_2$ et $l_1$, sont liées par les deux relations suivantes :

$$e_1 + h \leq l_1$$

$$e_1 + h + e_2 > l_1$$

Selon une autre caractéristique de la présente invention, les valeurs de $l_2$ et **p** sont liées par la relation suivante :

$$l_2 < p$$

D'autres caractéristiques et avantages de la présente invention apparaîtront au cours de la description suivante donnée en regard des dessins annexés à titre illustratif mais nullement limitatif, dans lesquels :

La figure 1 représente, sous forme schématique et vu en coupe longitudinale, un exemple de réalisation du système selon l'invention permettant de maintenir au moins deux portions d'os l'une par rapport à l'autre dans une application à une ostéosynthèse rachidienne,

La figure 2 représente une vue en coupe partielle d'une partie d'un autre mode de réalisation du système selon l'invention, et

La figure 3 représente une vue en coupe référencée III-III sur la figure 2.

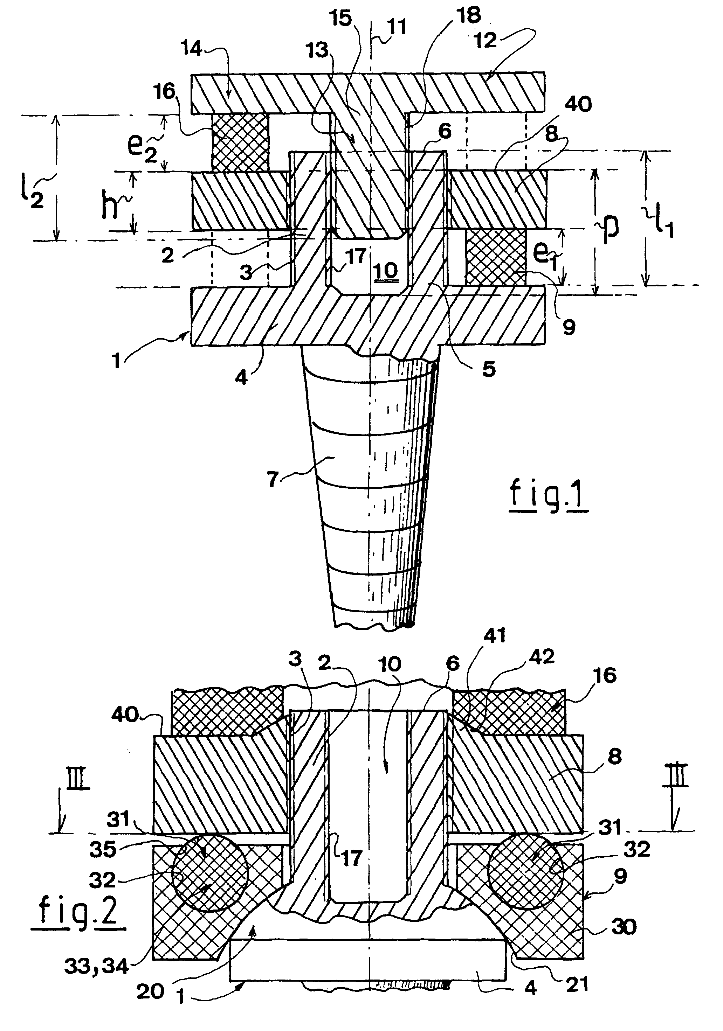

Les figures 1, 2 et 3 représentent deux modes de réalisation du système selon l'invention permettant de maintenir au moins deux portions d'os l'une par rapport à l'autre, dans une application à la réalisation d'une ostéosynthèse rachidienne. Cependant, dans le but de faciliter la compréhension de la présente description, les mêmes références y désignent les mêmes éléments, quelle que soit la figure sur laquelle elles apparaissent et quelle que soit la forme de représentation de ces éléments. De même, si des éléments ne sont pas spécifiquement référencés sur l'une des figures, leur référence peut être aisément retrouvée en se reportant à l'autre figure.

La Demanderesse tient à préciser que les figures ne représentent que deux modes de réalisation de l'objet selon l'invention et qu'il peut exister d'autres modes de réalisation qui répondent à la définition de cette invention.

Elle précise en outre que, lorsque, selon la définition de l'invention, l'objet de l'invention comporte "au moins un" élément ayant une fonction donnée, le mode de réalisation décrit peut comporter plusieurs de ces éléments.

Elle précise aussi que, si le mode de réalisation de l'objet selon l'invention tel qu'illustré comporte plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

Ceci ayant été précisé, la figure 1 représente un premier mode de réalisation simplifié d'un système permettant de maintenir au moins deux portions d'os l'une par rapport à l'autre, qui comporte une première vis 1 comportant une première tige 2 à filetage mécanique 3 et une première tête d'épaulement 4, cette première tige étant solidaire de la première tête d'épaulement par une première 5 de ses deux extrémités 5, 6, la longueur de cette première tige 2 étant égale à $l_1$, et des moyens 7 pour ancrer la première vis 1 dans une portion d'os, par exemple la partie osseuse d'une vertèbre. Ces moyens d'ancrage 7 peuvent être constitués, de façon classique, par une vis à filetage osseux solidaire par exemple de la tête d'épaulement 4. Une telle vis à filetage osseux est bien connue en elle-même et ne sera donc pas plus amplement décrite ici dans l'unique souci de simplifier la présente description, en précisant simplement que, dans ce cas, la tête d'épaulement de la vis à filetage osseux est constituée par la tête d'épaulement 4 de la première vis 1.

Le système d'ostéosynthèse comporte en outre un écrou 8 apte à se visser sur la première tige 2, la hauteur de cet écrou 8 étant égale à h, et au moins une première barre de liaison 9 apte à être maintenue par pincement entre l'écrou 8 et la première tête d'épaulement 4. Cette première barre de liaison 9 a une épaisseur $e_1$ et peut être de toute forme, une forme particulièrement avantageuse étant décrite ci-après en regard de la figure 2.

Est en outre prévu un orifice 10, par exemple borgne, réalisé sensiblement dans l'axe 11 de la première tige 2 et débouchant à la seconde extrémité 6 de cette première tige. Cet orifice 10 a une profondeur, suivant l'axe 11, égale à **p**, et sa paroi latérale comporte un filetage mécanique 17.

Le système permettant de maintenir au moins deux portions d'os l'une par rapport à l'autre comporte aussi une seconde vis 12 comprenant une seconde tige 13 à filetage mécanique 18 complémentaire du filetage mécanique 17 de la paroi latérale de l'orifice 10 défini ci-dessus et une seconde tête d'épaulement 14, cette seconde tige 13 étant solidaire de la seconde tête d'épaulement 14 par l'une 15 de ses deux extrémités, la longueur de cette seconde tige 13 étant égale à $l_2$, et au moins une seconde barre de liaison 16 apte à être maintenue par pincement entre l'écrou 8 et la seconde tête d'épaulement 14, l'épaisseur de cette seconde barre 16 étant égale à $e_2$.

De façon avantageuse, les valeurs **h**, $e_1$, $e_2$, et $l_1$ sont liées par les deux relations suivantes :

$$e_1 + h \leq l_1$$

$$e_1 + h + e_2 > l_1$$

De façon avantageuse également, les valeurs $l_2$ et **p** sont liées par la relation suivante :

$$l_2 < p$$

Le système permettant de maintenir au moins deux portions d'os l'une par rapport à l'autre défini ci-dessus, s'utilise de la façon suivante :

Lorsqu'un praticien veut réaliser une ostéosynthèse, par exemple entre deux vertèbres consécutives, il implante tout d'abord par exemple deux premières vis 1, chacune dans une partie osseuse dure d'une vertèbre, par vissage des moyens d'ancrage osseux 7, met en place un écrou 8 sur chaque première vis 1 en le vissant légèrement sur le filetage mécanique 3, puis introduit une seconde vis 12 dans chacune des premières vis, en la vissant partiellement dans l'orifice taraudé 10.

Si l'ostéosynthèse ne nécessite une liaison qu'entre deux vertèbres, il place une ou plusieurs barres de liaison 9 entre les écrous 8 et les premières têtes d'épaulement 4, puis il visse à fond les écrous 8 pour bien emprisonner les barres et les rendre solidaires des premières vis 1.

Si l'ostéosynthèse nécessite en revanche la mise en place de plusieurs barres de liaison suivant des directions différentes, comme par exemple illustré sur la figure 7 du FR-A-2 720 261, le praticien implante tout d'abord autant de premières vis 1 que nécessaire, visse légèrement sur chacune d'elles un écrou 8, puis une seconde vis 12 dans chaque orifice 10, et place au moins deux barres de liaison 9 et 16 respectivement entre les premières têtes d'épaulement 4 et les écrous 8, et entre les écrous 8 et les secondes têtes d'épaulement 14. Il oriente alors les barres de liaison pour obtenir le résultat curatif souhaité, puis visse à fond, tout d'abord les écrous 8 pour emprisonner les premières barres de liaison 9, et ensuite les secondes vis 12 pour enserrer la seconde barre de liaison 16 entre les secondes têtes d'épaulement 14 et la face 40 des écrous 8 opposée à celle qui est en contact avec la première barre de liaison 9.

Le système d'ostéosynthèse se présente alors sous la forme comme celle qui est schématiquement illustrée sur la figure 1, cette configuration étant obtenue, sans qu'il soit besoin de le développer ici, parce que les valeurs **h**, $e_1$, $e_2$, $l_1$, $l_2$ et **p** définies auparavant respectent les relations données ci-dessus.

Il est apparent que, quand le système ne comporte que des premières barres de liaison 9, la seconde vis 12 peut être vissée à fond dans l'orifice 10 de façon que sa tête d'épaulement 14 vienne au contact de l'extrémité 6 de la première tige 2. Le système n'est alors guère plus encombrant qu'avec seulement la première vis et l'écrou, puisqu'il ne déborde de cette première vis que de l'épaisseur de la tête d'épaulement 14. En revanche, cette tête d'épaulement 14, dont la face supérieure est avantageusement arrondie, permet de protéger les chairs du patient sur lequel a été implanté un tel système d'ostéosynthèse contre tout frottement sur les bords de l'orifice 10 et de l'écrou 8, et d'éviter des blessures. Ceci est l'un des avantages non négligeables présenté par le système selon l'invention permettant de maintenir au moins deux portions d'os l'une par rapport à l'autre.

Il est à noter que la configuration selon la figure 1 ne comporte qu'une seule barre de liaison entre la première tête d'épaulement 4 et l'écrou 8, et une seule entre l'écrou 8 et la seconde tête d'épaulement 14. Mais il est bien évident que le système peut comporter plusieurs barres en parallèle, par exemple deux barres 9 comme celle représentée en traits pleins et celle en traits interrompus, et deux barres 16 respectivement en traits pleins et en traits interrompus, de part et d'autre des tiges filetées 2 et 13. Mais il est aussi bien évident que ces barres peuvent être doublées, ou plus, par des barres parallèles et d'un même côté des tiges filetées, comme illustré par exemple sur les figures du FR-A-2 720 261 mentionné au préambule de la présente description.

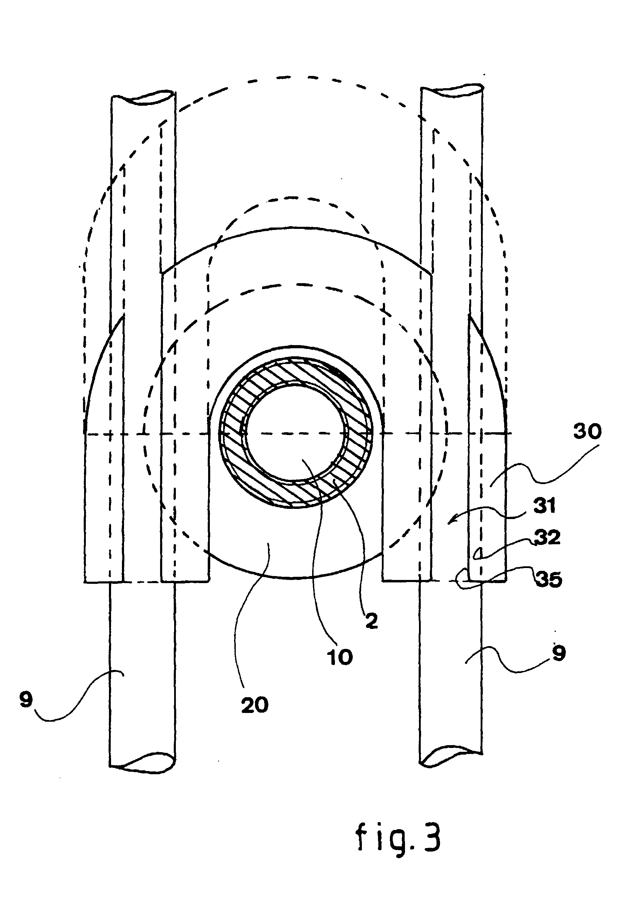

Les figures 2 et 3 représentent un autre mode de réalisation du système d'ostéosynthèse selon l'invention, pouvant constituer un perfectionnement à la réalisation selon la figure 1, étant précisé que ces figures 2 et 3 représentent des coupes partielles d'une partie du système d'ostéosynthèse, ne comportant pas la seconde vis 12.

Dans cette réalisation, la première tête d'épaulement 4 présente une surface d'épaulement 20 de forme hémisphérique et la première barre de liaison 9 comporte alors une surface de contact 21 complémentaire de cette surface d'épaulement de forme hémisphérique.

Cette réalisation permet plus facilement, au moins dans une certaine latitude, d'orienter spacialement la barre de liaison par rapport à la vis 1.

De plus, il est avantageux qu'au moins l'une des deux barres de liaison 9, 16 soit constituée par au moins un barreau 31, une embase 30 et un logement 32 de réception du barreau 31 réalisé dans l'embase. Cette réalisation étant particulièrement intéressante quand le barreau 31 est de forme sensiblement cylindrique de révolution, le logement 32 sera réalisé dans l'embase 30 de façon à être ouvert sur trois faces de cette embase, deux faces d'extrémités opposées et une face latérale, l'ouverture 35 sur la face latérale de l'embase ayant une largeur inférieure au diamètre du barreau cylindrique 31 et la section transversale de ce logement 32 étant complémentaire de celle du barreau 31 de façon que ce barreau puisse se positionner dans le logement 32 par glissement en étant introduit par l'ouverture 33, 34 du logement sur l'une des deux faces d'extrémités. Cette réalisation permet de positionner l'embase sur le barreau et de la faire coulisser le long de celui-ci, sans avoir à craindre qu'ils ne se désolidarisent. Elle facilite donc la pose d'une telle barre, ce qui est très avantageux notamment lorsque cette barre doit coopérer avec plusieurs vis 1 et/ou 12.

Pour faciliter la mise en place de tous les éléments constitutifs du système avant de procéder au serrage complet, il est particulièrement avantageux que l'embase 30 présente une forme sensiblement annulaire, ou semi-annulaire en fer à cheval comme représenté sur la figure 3, de façon à être apte à entourer au moins partiellement la première tige 2 tout en préservant un certain jeu pour pouvoir adapter, comme mentionné ci-dessus, l'orientation du barreau.

Dans une réalisation préférentielle, de façon à pouvoir encore mieux lier l'empilement quand l'ostéosynthèse nécessite l'utilisation des deux étages comme illustré sur la figure 1, il est avantageux que la face 40 de l'écrou 8 sur laquelle est apte à reposer la seconde barre 16 comporte une partie en saillie circulaire 41 de centrage, apte à coopérer avec une partie complémentaire en creux 42 réalisée sur la seconde barre 16.

Il est précisé que, avec un système selon le mode de réalisation illustré sur la figure 2, quand l'embase 30 a une forme annulaire fermée, les barres de liaison 9, 16 doivent être positionnées avant la mise en place, respectivement des écrous 8 et des secondes vis 12, contrairement au mode de réalisation selon la figure 1. En revanche quand l'embase 30 a une forme en fer à cheval comme illustré sur la figure 3, les barres de liaison 9 peuvent être positionnées après que l'écrou 8 ait été mis en place au moins partiellement. En effet l'embase peut alors être glissée le long des barres de liaison 9 jusqu'à ce qu'elle vienne au contact de la première tige de vis 2, comme représenté en traits continus sur cette figure 3, depuis une position éloignée comme celle représenté en traits interrompus sur cette même figure 3. L'écrou 8 est ensuite vissé définitivement pour fixer solidairement les barres et la vis 1.

A la description faite ci-dessus, apparaissent nettement tous les avantages du système selon l'invention permettant de réaliser une ostéosynthèse, notamment rachidienne, par rapport aux systèmes de l'art antérieur, notamment sa facilité d'utilisation et son encombrement réduit, même dans le cas où l'ostéosynthèse nécessite l'utilisation des deux étages comme illustré sur la figure 1.

## Revendications

1. Système permettant de maintenir au moins deux portions d'os l'une par rapport à l'autre, comportant :

   une première vis (1) comportant une première tige (2) à filetage mécanique (3) et une première tête d'épaulement (4), ladite première tige (2) étant solidaire de ladite première tête d'épaulement (4) par une première (5) de ses deux extrémités (5, 6), la longueur de cette première tige (2) étant égale à $l_1$,
   des moyens (7) pour ancrer la première vis (1) dans une portion d'os,
   un écrou (8) apte à se visser sur la première tige (2), la hauteur de cet écrou (8) étant égale à **h**,
   un orifice (10) réalisé sensiblement dans l'axe (11) de la première tige (2), ledit orifice (10) débouchant à la seconde extrémité (6) de la première tige (2) et ayant une profondeur égale à **p**, **caractérisé par** la paroi latérale dudit orifice comportant un filetage mécanique (17), et
   une seconde vis (12) comportant une seconde tige (13) à filetage mécanique (18) complémentaire du filetage mécanique (17) de la paroi latérale de l'orifice (10) et une seconde tête d'épaulement (14), ladite seconde tige (13) étant solidaire de ladite seconde tête d'épaulement (14) par l'une (15) de ses deux extrémités, la longueur de cette seconde tige (13) étant égale à $l_2$.

2. Système selon la revendication 1, **caractérisé par le fait que** les valeurs **h**, $l_1$, $l_2$ et **p** sont liées par les deux relations suivantes :

$$h \leq l_1$$

$$l_2 < p$$

**3.** Système selon la revendication 1, **caractérisé par le fait qu'**il comporte :

au moins une première barre de liaison (9) apte à être maintenue par pincement entre l'écrou (8) et la première tête d'épaulement (4), l'épaisseur de cette première barre de liaison (9) étant égale à $e_1$,

au moins une seconde barre de liaison (16) apte à être maintenue par pincement entre l'écrou (8) et la seconde tête d'épaulement (14), l'épaisseur de cette seconde barre (16) étant égale à $e_2$.

**4.** Système selon la revendication 3, **caractérisé par le fait que** les valeurs $h$, $e_1$, $e_2$ et $l_1$ sont liées par les deux relations suivantes :

$$e_1 + h \leq l_1$$

$$e_1 + h + e_2 > l_1$$

**5.** Système selon l'une des revendications 3 et 4, **caractérisé par le fait que** les valeurs de $l_2$ et **p** sont liées par la relation suivante :

$$l_2 < p$$

**6.** Système selon l'une des revendications 3 à 5, **caractérisé par le fait que** la première tête d'épaulement (4) présente une surface d'épaulement (20) de forme hémisphérique, ladite première barre (9) comportant une surface de contact (21) complémentaire de cette surface d'épaulement de forme hémisphérique (20).

**7.** Système selon l'une des revendications 3 à 6, **caractérisé par le fait qu'**au moins l'une des deux première et seconde barres de liaison (9, 16) est constituée par au moins un barreau (31), une embase (30) et un logement (32) de réception dudit barreau (31) réalisé dans ladite embase.

**8.** Système selon la revendication 7, **caractérisé par le fait que** ledit barreau (31) est de forme sensiblement cylindrique de révolution, ledit logement (32) étant réalisé dans l'embase (30) de façon à être ouvert sur trois faces de cette embase, deux faces d'extrémités opposées et une face latérale, l'ouverture (35) sur la face latérale de l'embase ayant une largeur inférieure au diamètre dudit barreau cylindrique (31), la section transversale de cedit logement (32) étant complémentaire de celle dudit barreau (31) de façon que ledit barreau puisse se positionner dans ledit logement (32) par glissement en étant introduit par l'ouverture (33, 34) dudit logement sur l'une des deux faces d'extrémités.

**9.** Système selon l'une des revendications 7 et 8, **caractérisé par le fait que** ladite embase (30) présente une forme sensiblement annulaire (fig. 2) apte à entourer ladite première tige (2).

**10.** Système selon l'une des revendications 7 et 8, **caractérisé par le fait que** ladite embase (30) présente une forme en fer à cheval (fig. 3).

**11.** Système selon l'une des revendications 3 à 10, **caractérisé par le fait que** la face (40) dudit écrou (8) sur laquelle est apte à reposer ladite seconde barre (16) comporte une partie en saillie circulaire (41) de centrage, apte à coopérer avec une partie complémentaire en creux (42) réalisée sur la seconde barre (16).

## Claims

**1.** A system enabling at least two bone portions to be held relative to each other, the system comprising:

a first screw (1) comprising a first shank (2) with a machine thread (3) and a first shoulder head (4), said first shank (2) being secured to said first shoulder head (4) via a first one (5) of its two ends (5, 6), the length of said first shank (2) being equal to $\ell_1$;
means (7) for anchoring the first screw (1) in a portion of bone;
a nut (8) suitable for screwing onto the first shank (2), the height of the nut (8) being equal to $\underline{h}$;
an orifice (10) formed substantially on the axis (11) of the first shank (2), said orifice (10) opening out in the second end (6) of the first shank (2) and having a depth equal to $\underline{p}$, the system being **characterized by** the side wall of said orifice including a machine thread (17); and
a second screw (12) comprising a second shank (13) having a machine thread (18) complementary to the machine thread (17) of the side wall of the orifice (10), and a second shoulder head (14), said second shank (13) being secured to said second shoulder head (14) via one (15) of its two ends, the length of said second shank (13) being equal to $\ell_2$.

**2.** The system of claim 1, **characterized by** the fact that the values $\underline{h}$, $\ell_1$, $\ell_2$, and $\underline{p}$ are associated by the following two relationships:

$$h \le \ell_1$$

$$\ell_2 < p$$

**3.** A system according to claim 1, **characterized by** the fact that it includes at least one connection bar (9) suitable for being held by being clamped between the nut (8) and the first shoulder head (4), the thickness of said first connection bar (9) being equal to $e_1$; and

at least one second connection bar (16) suitable for being held by clamping between the nut (8) and the second shoulder head (14), the thickness of said second bar (16) being equal to $e_2$.

**4.** A system according to claim 3, **characterized by** the fact that the values $\underline{h}$, $e_1$, $e_2$, and $\ell_1$ are related by the following two relationships:

$$e_1 + h \le \ell_1$$

$$e_1 + h + e_2 > \ell_1$$

**5.** A system according to claim 3 or claim 4, **characterized by** the fact that the values of $\ell_2$ and $\underline{p}$ are related by the following relationships:

$$\ell_2 < p$$

**6.** A system according to any one of claims 3 to 5, **characterized by** the fact that the first shoulder head (4) presents a shoulder surface (20) of hemispherical shape, said first bar (9) including a contact surface (21) complementary to said hemispherically-shaped shoulder surface (20).

**7.** A system according to any one of claims 3 to 6, **characterized by** the fact that at least one of the first and second connection bars (9, 16) is constituted by at least one rod (31), a base piece (30), and a housing (32) for receiving said rod (31) made in said base piece.

**8.** A system according to claim 7, **characterized by** the fact that said rod (31) is substantially in the form of a circular cylinder, said housing (32) being made in the base piece (30) in such a manner as to be open to three faces of said base piece, two opposite end faces and a side face, the opening (35) in the side face of the base being of a width that is smaller than the diameter of said cylindrical rod (31), the cross-section of said housing (32) being complementary to that of said rod (31) so that said rod can be positioned in said housing (32) by sliding, being inserted through the opening (33, 34) of said housing in one of the two end faces.

**9.** A system according to claim 7 or claim 8, **characterized by** the fact that said base piece (30) is substantially annular in shape (Figure 2) suitable for surrounding said first shank (2).

**10.** A system according to claim 7 or claim 8, **characterized by** the fact that said base piece (30) is horseshoe-shaped (Figure 3).

**11.** A system according to any one of claims 3 to 10, **characterized by** the fact that the face (40) of said nut (8) which is suitable for resting against said second bar (16) includes a circular projecting portion (41) for centering purposes that is suitable for cooperating with a complementary recess portion (42) formed in the second bar (16).

**Patentansprüche**

**1.** System, das ermöglicht, wenigstens zwei Knochenabschnitte zusammenzuhalten, und umfasst:

eine erste Schraube (1), die einen ersten Schaft (2) mit mechanischem Gewinde (3) und einen ersten Anschlagkopf (4) aufweist, wobei der erste Schaft (2) mit dem ersten Anschlagkopf (4) über ein erstes (5) seiner beiden Enden (5, 6) verbunden ist, wobei die Länge dieses ersten Schafts (2) gleich $l_1$ ist,
Mittel (7) zum Verankern der ersten Schraube (1) in einem Knochenabschnitt,
eine Mutter (8), die auf den ersten Schaft (2) geschraubt werden kann, wobei die Höhe dieser Mutter (8) gleich h ist,
eine Öffnung (10), die im wesentlichen längs der Achse (11) des ersten Schafts (2) verwirklicht ist, in das zweite Ende (6) des ersten Schafts (2) mündet und eine Tiefe besitzt, die gleich p ist, **dadurch gekennzeichnet, dass** die Seitenwand der Öffnung ein mechanisches Gewinde (17) aufweist, und
eine zweite Schraube (12) vorgesehen ist, die einen zweiten Schaft (13) mit mechanischem Gewinde (18), das zu dem mechanischen Gewinde (17) der Seitenwand der Öffnung (10) komplementär ist, sowie einen zweiten Anschlagkopf (14) aufweist, wobei der zweite Schaft (13) mit dem zweiten Anschlagkopf (14) über eines (15) seiner beiden Enden verbunden ist, wobei die Länge dieses zweiten Schafts (13) gleich $l_2$ ist.

2. System nach Anspruch 1, **dadurch gekennzeichnet, dass** die Werte h, $l_1$, $l_2$ und p durch die beiden folgenden Beziehungen miteinander verbunden sind:

$$h \leq l_1$$

$$l_2 < p.$$

3. System nach Anspruch 1, **dadurch gekennzeichnet, dass** es umfasst:

wenigstens ein erstes Verbindungsglied (9), das durch Klemmung zwischen der Mutter (8) und dem ersten Anschlagkopf (4) gehalten werden kann, wobei die Dicke dieses ersten Verbindungsglieds (9) gleich $e_1$ ist, und wenigstens ein zweites Verbindungsglied (16), das durch Klemmung zwisehen der Mutter (8) und dem zweiten Anschlagkopf (14) gehalten werden kann, wobei die Dicke dieses zweiten Glieds (16) gleich $e_2$ ist.

4. System nach Anspruch 3, **dadurch gekennzeichnet, dass** die Werte h, $e_1$, $e_2$ und $l_1$ durch die beiden folgenden Beziehungen miteinander verbunden sind:

$$e_1 + h \leq l_1$$

$$e_1 + h + e_2 > l_1.$$

5. System nach einem der Ansprüche 3 und 4, **dadurch gekennzeichnet, dass** die Werte von $l_2$ und p durch die folgende Beziehung miteinander verbunden sind:

$$l_2 < p.$$

6. System nach einem der Ansprüche 3 bis 5, **dadurch gekennzeichnet, dass** der erste Anschlagkopf (4) eine halbkugelförmige Anschlagfläche (20) aufweist, wobei das erste Verbindungsglied (9) eine zu dieser halbkugelförmigen Anschlagfläche (20) komplementäre Kontaktfläche (21) aufweist.

7. System nach einem der Ansprüche 3 bis 6, **dadurch gekennzeichnet, dass** wenigstens eines der ersten und zweiten Verbindungsglieder (9, 16) durch wenigstens einen Stab (31), einen Sockel (30) und einen in dem Sockel verwirklichten Aufnahmesitz (32) für die Aufnahme des Stabs (31) gebildet ist.

8. System nach Anspruch 7, **dadurch gekennzeichnet, dass** der Stab (31) eine im wesentlichen rotationssymmetrische zylindrische Form hat, wobei der Aufnahmesitz (32) in dem Sockel (30) in der Weise verwirklicht ist, dass er an drei Seiten dieses Sockels, d. h. an zwei gegenüberliegenden Stirnflächen und an einer Seitenfläche, offen ist, wobei die Öffnung (35) zu der Seitenfläche des Sokkels eine Weite besitzt, die kleiner als der Durchmesser des zylindrischen Stabs (31) ist, wobei der Querschnitt dieses Aufnahmesitzes (32) zu jenem des Stabs (31) komplementär ist, so dass der Stab in dem Aufnahmesitz (32) gleitend angeordnet werden kann, indem er durch die Öffnung (33, 34) des Aufnahmesitzes an einer der beiden Stirnseiten eingeführt wird.

9. System nach einem der Ansprüche 7 und 8, **dadurch gekennzeichnet, dass** der Sockel (30) im wesentlichen ringförmig ist (Fig. 2) und den ersten Schaft (2) umgeben kann.

10. System nach einem der Ansprüche 7 und 8, **dadurch gekennzeichnet, dass** der Sockel (30) hufeisenförmig ist (Fig. 3).

11. System nach einem der Ansprüche 3 bis 10, **dadurch gekennzeichnet, dass** die Fläche (40) der Mutter (8), auf der das zweite Glied (16) aufliegen kann, einen kreisförmigen, vorstehenden Zentrierabschnitt (41) aufweist, der mit einem an dem zweiten Stab (16) verwirklichten komplementären Hohlabschnitt (42) zusammenwirken kann.

fig.1

fig.2

fig. 3